Europäisches Patentamt

European Patent Office

Office européen des brevets

(19)

(11) Publication number: **0 146 074**
**B1**

(12) **EUROPEAN PATENT SPECIFICATION**

(45) Date of publication of patent specification: **10.08.88**

(51) Int. Cl.⁴: **A 61 B 5/14,** B 01 L 3/14

(21) Application number: **84114725.9**

(22) Date of filing: **04.12.84**

(54) Blood collection device.

(30) Priority: **05.12.83 US 557962**

(43) Date of publication of application:
**26.06.85 Bulletin 85/26**

(45) Publication of the grant of the patent:
**10.08.88 Bulletin 88/32**

(84) Designated Contracting States:
**DE FR GB NL SE**

(56) References cited:
**EP-A-0 072 006**
**DE-A-3 201 691**
**FR-A-2 173 661**

**PATENTS ABSTRACTS OF JAPAN, vol. 6, no.
237 (P-157)1115r, 25th November 1982; & JP -
A - 57 136 166 (TATEISHI DENKI K.K.) 23-08-
1982**

(73) Proprietor: **Terumo Medical Corporation**
**125 Blue Ball Road**
**Elkton Maryland 21921 (US)**

(72) Inventor: **Proud, Todd A.**
**719 Elmtree Lane**
**Claymont Delaware 19703 (US)**
Inventor: **Mitchell, Wayne J.**
**270 Greenwood Street**
**Elkton Maryland 21921 (US)**
Inventor: **Lin, Tom F.**
**24 Savanah Drive East**
**Bear Delaware 19701 (US)**
Inventor: **Kasper, James D.**
**Rt 3**
**Montpelier Ohio 43543 (US)**

(74) Representative: **Hänzel, Wolfgang, Dipl.-Ing.
et al
Patentanwälte Henkel, Pfenning, Feiler, Hänzel,
Meinig Möhlstrasse 37
D-8000 München 80 (DE)**

Courier Press, Leamington Spa, England.

## Description

### Field of the Invention

This invention relates to a blood collection device. More particularly, it relates to a blood collection device designed to collect a minute amount of blood, so called capillary blood from capillary blood vessels in a punctured skin formed in an earlobe, a finger tip or a heel.

### Description of the Prior Art

In the recent years, the technique of clinical analysis has remarkably advanced and reached a point where various hematologic diagnoses can be performed with a minute amount of blood. It is no longer necessary to collect a large amount of blood from the vein of a patient. Instead, it now suffices to form a puncture in the skin of the patient's earlobe, finger tip or heel and collect a very small amount of peripheral blood exuding from the capillary blood vessels in the punctured skin. This method of blood collection is mainly adopted particularly when the vein is very thin as in a newborn baby so that the syringe used for blood collection cannot be easily plunged into the vein. To work this method of blood collection effectively, it becomes necessary to use a device capable of collecting the blood before the blood has a chance to clot.

Heretofore, the collection of capillary blood by this method has been effected by virtue of the capillarity produced with a capillary tube of glass. Where the capillarity is utilized, there ensues a possiblity that whenm the exudation of blood is retarded or interrupted, the blood rising in the capillary tube will impede smooth flow of blood after the exudation is resumed by existence of air in the blood. At the time of actual blood collection this method requires the clinician to be accustomed to the technique of quickly and accurately disposing the leading end of the capillary tube at the punctured skin of the patient. Where the quick and accurate disposition of the capillary tube is not obtained, the collection of blood by capillarity is not started or it is retarded. As the result, the capillary blood is suffered to clot. Further, when the blood is collected by using such glass capillary having small diameter, it was difficult to remove serum which is separated as upper layer of the capillary after centrifuging the collected blood.

To overcome these disadvantages, capillary blood collection devices which obviate the necessity for capillarity have been developed. For example, the scoop type minute blood collection device disclosed by Japanese Patent Laid-open SHO 58(1983)—29,451 is one of such devices. This scoop type minute blood collection device comprises a collection container body and a cap fitted on the collection container body and provided with a blood passage having a semitubular blood collection member (scoop) formed at the leading end thereof and serving to introduce blood and an air passage separated from the blood passage with a partition wall and intended to make up for insufficient displacement of air with blood effected withing the blood passage of a small diameter. The scoop type minute blood collection device constructed as described above necessitates provision of an extra member adapted to keep the opening of the collection container body closed during the storage of the devices or after blood collection for the purpose of preventing the interior of the collection container body from exposure to the ambient atmosphere. Prior to blood collection, this extra member is removed from the opening of the collection container body and replaced with the aforementioned cap. This device, therefore entails troublesome handling before its actual use. Since the device is composed of three components, i.e. a cap of complicated shape, a collection container body, and a closure member, it suffers from high cost of production. Although the capillary blood is collected without resorting to any capillary action, the blood is admitted into the collection container body via an annular blood passage of a considerably smaller diameter than the collection container body. If the annular skirt forming the blood passage is not intimately joined with the inner wall surface of the collection container body, therefore, there arises a possibility that the blood of relatively high viscosity will be caused by surface tension to form a film and consequently drops within the blood passage so much as to interrupt the flow of blood into the collection container body. There are times when the collection container has various reagents to the intended test items contained in advance therein. For the identification of such reagents, the practice of placing a marking such as a color mark according with an established code on the aforementioned closure member which has no possibility of impairing the transparency of the collection container body is widely followed, because plural blood collection body respectively contained different reagents are used at the same time at the blood test. In the case of the minute blood collection device constructed as described above, however, the closure member is separated from the collection container body prior to punctual a blood collecting part of the patient in order to collect blood. Once the closure member is so separated, the identifying mark by color code elaborately placed thereon often fails to serve the purpose of identification through confusion of the particular closing member with the blood collection container body. If the reagents in the body is not identified, it is dangerous because wrong results are obtained.

Prior art document FR—A—2,173,661 discloses a blood collection device, which comprises a blind tubular container having an open upper end portion and a second lower end portion. A collection portion is disposed at the upper end portion of the container and projects upwardly from the upper end portion of the container. The blood is to the interior of the container, and a closure member is provivided with a depression of such shape and size as to be optionally fitted to the lower end portion of the container and adapted to close the open upper end portion of the container.

Further, it is known from JP—A—57—136,166, for example, to provide a closure member of a sample containing device with indications for visual identification or from DE—A—3,201,691 to provide a closure member for a blood collection device with wiping means adapted to slide on the inner surface of the collection portion in direction to the lower end part of a container. The closure member has further an external sleeve which surrounds the wiping means with a gap therebetween. The width of this gap corresponds substantially to the wall thickness of the blood collection device which is tightly closed when its upper edge is inserted into the gap.

It is an object of the present invention to provide a blood collection device pre-assembled to be readily put to use and enabled to keep the interior thereof from exposure to the ambient atmosphere. A further object of this invention is to provide a blood collection device which enjoys ease of operation and handling and has virtually no possibility of the sample blood clotting during the course of blood collection. Still another object of this invention is to provide a blood collection device which permits easy identification of reagents placed in advance therein. Yet another object of the present invention is to provide a blood collection device which permits easy removal of serum subsequent to centrifugal separation of the contained blood.

The present invention provides a blood collection device comprising a blind tubular container portion having an open upper end portion and a closed lower end portion, a collection portion being disposed at the upper end portion of said container portion and projecting upwardly from the upper end portion of said container portion and having means for guiding the blood to the interior of said container portion, a closure member provided with a depression of such shape and size as to be releasably fitted to the lower end portion of said container portion and removable therefrom in order to close the upper end portion of said container portion, wherein one portion of said container portion has a diameter which gradually increases upwardly, said closure member is provided with a covering lid and with plug means adapted to slide tightly on the inner surface of said collection portion downwardly towards the lower end part of the container portion, said plug means has a smaller diameter than that of the covering lid, is separated from said covering lid by an annular groove and extends downwardly coaxially with said lid, the collection portion comprises an edge which fits in the annular goove and enables said covering lid to serve as a protective cover for an outer part of said edge, and said plug has a hollow interior and an open upper end to form said depression, said blood collection device being characterized in that the collection portion and the container portion are integrally formed, said edge is arcuate and projects from the circumference of the upper end of the container portion. The closure member comprises an indication for enabling visual identification.

The blood collection device of the present invention possesses in integral combination a collector and a container which jointly function to collect and store the capillary blood flowing out of the punctured skin of the patient. The closure member is used for tightly closing the aforementioned integral combination of collector and container before and after sample collection. The closure bears and identifying color mark according with the established color code. Further, this closing member is so constructed that, during the collection of sample blood, the bottom of the aforementioned integral combination of collector and container will be kept fitted in the top of the aforementioned closure member.

Preferred embodiments of the invention are explained with reference to the drawings in which

Fig. 1 is a partially cutaway front view of a typical capillary blood collection device as one embodiment of this invention,

Fig. 2 is a plan view of the device of Fig. 1,

Fig. 3 is a side view illustrating a collector combination and a closure member of the aforementioned device,

Fig. 4 is a plan view illustrating the collector-container combination of the device as fitted in the closure member, and

Fig. 5 is a cross section of the device of Fig. 4 taken along the line V—V, illustrating the device in a state held during the collection of sample blood.

Description of Preferred Embodiment

Now, the present invention will be described in detail below with reference to the accompanying drawings illustrating a preferred embodiment.

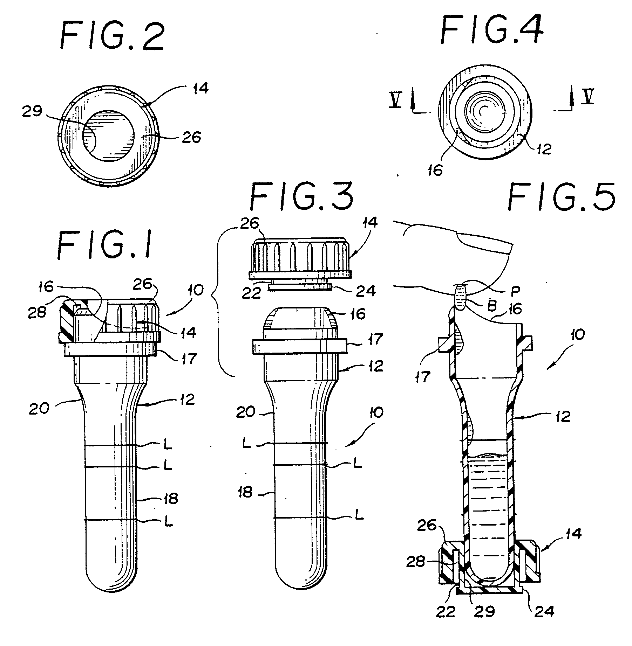

Fig. 1 through Fig. 4 illustrates a blood collection device 10 of this invention. As noted from these diagrams, the blood collection device 10 comprises two members, i.e. an integral combination of collector and container (hereinafter referred to as "collector-container") 12 and a closure member 14. In the collector-container 12, a collection portion 16 and a container portion 18 are only requred to be jointly in a state which permits flow of a fluid. Preferably for the purpose of ensuring smooth connection of the collection portion 16 and the container portion 18 and avoiding useless complication of shape, the interior of the collection portion 16 and that of the container portion 18 are formed of one and the same curved surface. The collection-container 12 is desired to be an integral member molded of synthetic resin. The collection portion or top portion 16 is provided along the periphery thereof with an upwardly swelled arcuate edge. As illustrated in Fig. 5, this arcuate edge functions as a blood collector for effectively conveying the blood B from the position P of punctured skin to the container 18. Further, the collection portion 16 is desirably provided on the outer peripheral surface thereof with an annular rib 17, which functions as a contact surface for the closure member 14 at the time that the closure member is fitted in the collection portion 16 and as a stopper at the time that the device is inserted in a holder such as of a centrifugal separator, for example. In the meantime, the bottom of the container portion 18 is generally in the shape of a blind tube having a rounded tip or a hemispherical bottom. The container portion 18 is generally provided closed bottom and an upwardly flared open upper end 20. The collection portion 16 which is integrally connected to the upwardly flared upper end 20, therefore, is allowed to have a relatively large diameter. Because the blood B being collected is not suffered to spill out of the blood collection device 10 and the air inside the container portion 18 is easily displaced with the blood B, the blood B is smoothly conveyed via the collection portion 16 to the container portion 18. On the other hand, the lower section of the container portion which has a relatively small diameter because of the gradual convergence from the upper end 20 renders the container portion suitable as a blood centrifugal portion. When the blood is centrifuged, it is divided into an upper layer formed of serum and a lower layer formed of cruor. This layer of serum is required to possess a certain extent of depth in order to enhance to remove from the blood collection device. Since the lower section of the container portion possesses a relatively small diameter, the layer of serum held in that section is allowed to have a depth exceeding the perscribed level compared with one possessing larger diameter in case of the same volume. The container portion 18 can be filled with the blood to the prescribed level L. Optionally, container portion 18 may have such additives as an anticoagulant for blood, a blood coagulation aid and/or a gel substance incorporated therein having specific gravity intermediate the specific gravity of the phases of blood to be separated, for example, prior to blood collection.

In a preferred embodiment of this invention, the closure member 14 serves as a site for visual identification with color marks according with the established color code. Thus, closure members bearing dissimilar color marks are distributed to collector-containers containing additives corresponding to the color marks or non-containing additives. With a glance at the color marks, the user is enabled to know immediately what additives the collector-containers placed before him are containing or non-containing. Indication of kinds of the blood collection device is limited not only by color code but also by letter and the like indicated on the closure member. Preferably, the closing member 14 is provided with a tubular plug 22 exteded downwardly in the axial direction so as to fit snugly on the collection portion 16 of the blood collection device 10 as illustrated in Figs. 1 and 3 and is molded of a material. In such a manner of use as described above, the plug 22 may have the same diameter through the upper and lower portions which slides tightly with inner surface of the collection portion, preferably the plug 22 formed so as to incorporate an elastic annular flange 24 functions as an effective tight sealer for the open upper end of the collector-container 12. As illustrated more clearly in Figs. 3 and 5, the plug 22 is extended so as to define an annular groove 28 between the outer face of the plug and the inner face of the covering lid or main portion 26. When the closure member 14 functions as a tight sealer, the collection portion 16 intimately fits into the groove 28 and the covering lid 26 forms an effective cover for the collection portion 16. In this manner, the interior of the collector-container 12 is tightly closed with the flange 24 and, at the same time, the outer face of the collection portion 16 is covered with the covering lid 26. Thus, the collector-container 12 is effectively sealed during transportation and storage and the additives held inside the container portion is safely preserved until sample collection.

Prior to the blood collection, the closure member 14 is removed from the open top of the collector-container 12 and the bottom of the container portion 18 is fitted into the hollow interior 29 of the plug 22 of the closure member as illustrated in Fig. 5. This means that the closure member 14 accompnaies the collector-container 12 at all times. When the closure member is utilized as the site for visual identification with a color mark and the like, therefore, the user is enabled to tell at any time what additives the container portion 18 of the collector-container 12 is holding therein or non-additive.

The closure member 14 fulfils several functions other than the aforementioned function to keep the collector-container tightly sealed during the blood collection and permit identification of the additives contained therein. For example, when sample is collected and the closure member 14 is fitted on the open top of the collector-container 12, the plug 22 plays the portion of a wiper for rubbing the serum remaining in the collection portion 16 into the container portion 18. Even after the sample blood collection, the closure member 14 continued to function as a tight sealer.

The blood collection device 10 is put to use as follows. The form of blood collection device 10 prior to use is illustrated in Fig. 1. The closure member 14 is removed and the bottom of the collector-container 12 is fitted into the top of the closure-member 14 as illustrated in Fig. 5. At the time that the blood collection is started, the capillary blood collection device is held upright, and the edge of the collection portion 16 is arcuated so as to guide and convey the capillary blood B easily into the collection portion 16. The blood B flows through the collection portion 16 and drops under its own weight into the container portion 18. This transfer of the blood continues until there is obtained an ample blood sample. Then, the closure member 14 is removed from the bottom of the container part 18 and disposed on the collection portion 16 to provide effective tight seal for the collector-container 12. In such case, the periphery of the collection portion 16 serves to guide the closure member 14 and enhance to fit. The content of the container portion 18 is blended with the additives such as coagulating agents or coagulation aids by gently rotating the capillary blood collection device eight to ten times, for example. Consequently, there is obtained a specimen in a state ready for transfer to a laboratory for clinical test.

Preferably, the capillary blood collection device 10 is provided in a preassembled state so as to avoid pollution of an interior by air. The components which make up the blood collection device 10 can be easily formed of a moldable material such as transparent plastic and/or rubber inactive to blood testing reagents by any of the methods known to the art.

Optionally, in the blood collection device 10 of the present invention, a hydrophilic treating may be conducted on the inner wall surface of the collector-container 12 so as to enhance the smooth flow of the blood B on the inside of the collector-container 12. By such hydrophilic treating, mixing of blood with the additive can be easily conducted. The hydrophilic treating may be conducted, for example, by plasma discharging or by coating polyethylene glycol-dimethyl polysiloxane copolymer (e.g., SH 3771; Toray Silicone Co., Ltd.).

Since this invention provides a blood collection device which, as described above, comprises a blind tubular container portion having an open upper end portion and a closed lower end portion, a collection portion disposed at the upper end portion of the container portion, projected upwardly from the upper end portion of the container portion, and having means for guiding the blood to the interior of the container portion, and a closure member provided with a depression of such shape and size as to be optionally fitted to the lower end portion of the container portion and adapted to closable the open upper end portion of the container portion, and blood exuded from the vessels exposed in the punctured skin of the patient can be easily collected and stored. When the closure member is selected to be used as the site for visual identification by use of a color mark according with the established color code, the additives placed in advance in the container portion of the blood collection device can be identified at all times.

Alternatively when there is no additive in the device, it also can be easily found. Where a plurality of blood collection devices containing different kinds of additives are handled all at once, these color marks on the closure members preclude the possibility of the contents of the container portions being confused one with another after blood collection. When the closure member is provided with the plug incorporating therein wiping means adapted to wipe the inner face of the collection portion at the time that the closing member is fitted on the collection portion, the serum adhering to the terminal portion can be made to move into the container portion. Thus, the possibility that the blood remaining within the collection portion will not contaminate the serum by mixing during the centrifugal separation and consequently disturb the separated layers is precluded.

All these effects of the present invention will be improved further by modifying the blood collection device so that a collection portion and a container portion are integrally formed, the upper end of the collection part is formed to constitute an arcuate edge destined to serve as means for guide conveying blood, a closure member comprises a covering lid and a plug separated from the covering lid by an annular groove and extended downwardly coaxially with the lid, the lid is therefore capable of tightly closing the collection portion, the aforementioned edge fits in the annular groove and consequently enables the covering lid to serve as a protective cover for the edge and to prevent contamination while the device is not in use for blood collection, and the plug is formed in a hollow shape having an open upper end used for the formation of the aforementioned depression. Further when the capillary blood collection device is so constructed that the upper end section of the container portion of the relatively small diameter is gradually diverged upwardly after the pattern of a flare and finally connected integrally to the collection portion of the relatively large diameter, the collection of blood can be carried out more easily and safely and the withdrawal of the serum after centrifugal separation of the blood can be effected easily.

## Claims

1. A blood collection device comprising
— a blind tubular container portion (12) having an open upper end portion and a closed lower end portion,
— a collection portion (16) being disposed at the upper end portion of said container portion (12) and projecting upwardly from the upper end portion of said container portion (12) and having means for guiding the blood to the interior of said container portion (12),
— a closure member (14) provided with a depression (29) of such shape and size as to be releasably fitted to the lower end portion of said container portion (12) and removable therefrom in order to close the upper end portion of said container portion (12), wherein
— one portion (20) of said container portion (12) has a diameter which gradually increases upwardly,
— said closure member (14) ia provided with a covering lid (26) and with plug means (22) adapted to slide tightly on the inner surface of said collection portion (16) downwardly towards the lower end part of the container portion (12),
— said plug means (22) has a smaller diameter than that of the covering lid (26), is separated from said covering lid (26) by an annular groove (28) and extends downwardly coaxially with said lid (26),
— the collection portion (16) comprises an edge which fits in the annular groove (28) and enables said covering lid (26) to serve as a protective cover for an outer part of said edge, and

— said plug (22) has a hollow interior and an open upper end to form said depression (29), characterized in that

the collection portion (16) and the container portion (12) are integrally formed and said edge is arcuate and projects from the circumference of the upper end of the container portion (12).

2. A blood collection device according to claim 1, characterized in that said closure member (14) has thereon an indication of the contents of said blood collection device for visual identification.

**Patentansprüche**

1. Blutentnahmevorrichtung, umfassend

— einen (einseitig) verschlossenen Behälterabschnitt (12) mit einem offenen oberen Endabschnitt und einem geschlossenen unteren Endabschnitt,

— einen am oberen Endabschnitt des Behälterabschnitts (12) angeordneten Sammel- oder Abnahmeabschnitt (16), der vom oberen Endabschnitt des Behälterabschnitts (12) nach oben absteht und Mittel zum Leiten des Bluts in das Innere des Behälterabschnitts (12) aufweist,

— ein Verschlußelement (14), das mit einer Ausnehmung (29) einer solchen Form und Größe versehen ist, daß es abnehmbar am unteren Endabschnitt des Behälterabschnitts (12) anbringbar und von ihm entfernbar ist, um den oberen Endabschnitt des Behälterabschnitts (12) zu verschließen, wobei

— ein Abschnitt (20) des Behälterabschnitts (12) einen Durchmesser aufweist, der sich in Aufwärtsrichtung allmählich oder fortlaufend vergrößert,

— das Verschlußelement (14) mit einer Abschlußkappe (26) mit einem Stopfenmittel (22) versehen ist, das mit engem Sitz auf der Innenfläche des Abnahmeabschnitts (16) abwärts in Richtung auf den unteren Endteil des Behälterabschnitts (12) verschiebbar ist,

— das Stopfenmittel (22) einen kleineren Durchmesser als die Abschlußkappe (26) aufweist, von letzterer durch eine Ringnut (28) getrennt ist und sich in der Kappe (26) koaxial (dazu) abwärts erstreckt,

der Abnahmeabschnitt (16) eine Randkante aufweist, die in die Ringnut (28) einpaßbar ist und die Abschlußkappe (26) als Schutzabdeckung für einen Außenteil der Randkante wirken läßt, und

— der Stopfen (22) einen hohlen Innenraum und ein offenes oberes Ende, welche die Ausnehmung (29) bilden, aufweist,

dadurch gekennzeichnet, daß

der Abnahmeabschnitt (16) und der Behälterabschnitt (12) materialeinheitlich ausgebildet sind und die Randkante gekrümmt geformt ist und vom Umfang des oberen Endes des Behälterabschnitts (12) vorsteht.

2. Blutabnahmevorrichtung nach Anspruch 1, dadurch gekennzeichnet, daß am Verschlußelement (14) ein Anzeigemittel für den Inhalt der Blutabnahmevorrichtung für visuelle Bestimmung desselben vorgesehen ist.

**Revendications**

1. Dispositif pour prélèvement de sang, comportant

— une partie (12) formant récipient tubulaire aveugle, ayant une partie extrême supérieure ouverte et une partie extrême inférieure fermée,

— une partie (16) pour prélèvements, disposée au niveau de la partie extrême supérieure de ladite partie (12) formant récipient et faisant saillie vers le haut depuis la partie extrême supérieure de ladite partie (12) formant récipient et ayant un moyen pour guider le sang jusqu'à l'intérieur de ladite partie (12) formant récipient,

— un élément de fermeture (14) pourvu d'une dépression (29) ayant un profil et des dimensions telles qu'il peut être installé de manière amovible sur la partie extrême inférieure de ladite partie (12) formant récipient, et pouvant être retiré de celle-ci afin de fermer la partie extrême supérieure de ladite partie (12) formant récipient, dans lequel

— une portion (20) de ladite partie (12) formant récipient a un diamètre qui augmente progressivement vers le haut,

— ledit élément de fermeture (14) est pourvu d'un couvercle couvrant (26) et d'un moyen formant bouchon (22) conçu pour coulisser vers le bas tout contre la surface interne de ladite partie (16) pour prélèvements, vers la partie extrême inférieure de la partie (12) formant récipient,

— ledit moyen formant bouchon (22) a un diamètre plus petit que celui du couvercle couvrant (26), est séparé dudit couvercle couvrant (26) par une gorge annulaire (28) et s'étand vers le bas, coaxialement avec ledit couvercle (26),

— et la partie (16) pour prélèvements comporte un bord qui s'ajuste dans la gorge annulaire (28) et permet audit couvercle couvrant (26) de servir de pièce de fermeture protectrice pour une partie extérieure dudit bord, et

— ledit bouchon (22) est intérieurement creux et a une extrémité supérieure ouverte pour former ladite dépression (29),

caractérisé en ce que

la partie (16) pour prélèvements et la partie (12) formant récipient sont formées d'une seule pièce, et ledit bord est arqué et fait saillie depuis la circonférence de l'extrémité supérieure de la partie (12) formant récipient.

2. Dispositif pour prélèvement de sang selon la revendication 1, caractérisé en ce que ledit élément de fermeture (14) porte sur lui une indication relative au contenu dudit dispositif pour prélèvement de sang, à des fins d'identification visuelle.

FIG.2

FIG.4

FIG.3

FIG.5

FIG.1